# EUROPEAN PATENT SPECIFICATION

(11) **EP 1 075 843 B1**
(45) Date of publication and mention of the grant of the patent: **02.02.2005**
(21) Application number: 99938676.6
(22) Date of filing: 29.07.1999
(51) Int. Cl.: A61L 17/00, A61B 17/06

(54) **SURGICAL THREAD FOR PLASTIC SURGERY OPERATIONS**
CHIRURGISCHER FADEN FÜR OPERATIONEN IN DER PLASTISCHEN CHIRURGIE
FIL CHIRURGICAL POUR OPERATIONS DE CHIRURGIE PLASTIQUE

(30) Priority: 03.03.1999 RU 99103732
(43) Date of publication of application: 14.02.2001
(73) Proprietor: Sulamanidze, Marlen Andreevich, Moscow, 115304 (RU); Mikhailov, Georgy Marlenovich, Moscow, 115304 (RU)
(72) Inventor: Sulamanidze, Marlen Andreevich, Moscow, 115304 (RU); Mikhailov, Georgy Marlenovich, Moscow, 115304 (RU)
(74) Representative: Hano, Christian, Dipl.-Ing.
(86) International application number: PCT/RU1999/000263
(87) International publication number: WO 2000/051658

(56) References cited:
- EP-A- 0 576 337
- WO-A-98/52473
- DE-A- 1 810 800
- DE-A1- 4 302 895
- GB-A- 1 506 362
- US-A- 3 123 077
- US-A- 5 222 976
- US-A- 5 374 268

## Description

This invention relates surgical threads for use in plastic surgery operations and particularly, but not exclusively, cosmetic plastic surgery operations.

Surgical threads are well known and may be made from different materials: metallic, polymeric, biological. Traditionally, such threads need strength, a smooth surface, bioinertia, the ability to be tied in knots, and resistance to elongation. Metallic threads may be made of tantalum, silver, nickel and other wires. Non-metallic threads may be made of lavsan, nylon, caprone, polypropylene, vikril, polysorb, etc. The threads may be monolithic, wattled or twisted.

Conventional surgical threads can be pulled through soft tissues, for example skin and hypodermic tissue, along their whole length, in either direction. This is a disadvantage during some plastic surgery operations. While suturing a wound by an intradermic or subcuticular continuous suture, in order to strengthen the ends of the thread at the beginning and end of the wound it is necessary to suture the wound with an interrupted suture or to plaster the patches. This lengthens the operation time and makes difficult the process of removing the suture; sometimes due to the instability of strengthening of the edges of a wound part.

Known methods of suturing include interrupted, mattress and continuous. While performing continuous intradermic suturing with smooth threads, usually a suture is smoothed out along the length of the wound's line. This results in a maximum length of scar, which is aesthetically undesirable. If during the suturing of a wound, the skin is uniformly gathered along the line of the wound, it is possible to obtain a shorter scar. However, when using smooth threads it is impossible to obtain uniform corrugation of the wound's line along its length: the skin is more gathered at the ends, being more smoothed out close to the centre, resulting in necrosis of more gathered tissues and an unfavorable form of scar.

It is known to modify a conventional surgical thread so that it easily penetrates tissue in only one direction. For example, patent document DE-A-4302895 describes a surgical thread with inclined protrusions arranged sequentially along its length with the ends of the protrusions oriented in a direction away from the needle end of the thread. The other end of the thread has a an eyelet through which the needle-end of the thread can be passed to form the thread into a loop without the need for a knot. This type of thread would not be suitable for continuous suturing.

This invention is more particularly concerned with a surgical thread having a needle at one end thereof and a sequence of protruding, conical barbs with sharp ends arranged along its length, a first plurality of the barbs being inclined in a direction away from the needle-end of the thread to permit the thread to be drawn by the needle through a patient's tissue but resist sliding of the thread through the tissue in the opposite direction, and a second plurality of the barbs being inclined in a direction towards the needle-end of the thread.

Patent documents DE-A-1810800 and WO-A-98/52473 each describe such a surgical thread having needles at both ends. Barbs along one half of the thread are oriented away from the needle at the end of that half of the thread, and barbs along the other half of the thread are oriented away from the needle at the end of that other half of the thread. When joining two pieces of tissue, each needle can be inserted into a respective piece of the tissue, and half of the thread can be pulled into that piece of tissue. The barbs resist sliding of the thread back through the tissue so that the tissue is held together at the join. DE-A-1810800 also describes a modification in which one of the needles is omitted and the thread is sufficiently stiff that is can be pushed endwise into a sinew. Again, all of these threads are unsuitable for continuous suturing. WO-A-98/52493 corresponds to the preamble of claim 1.

The aim of the invention is to provide such a surgical thread that is better suited to cosmetic plastic surgery operations and that can be more readily used for continuous intradermic or subcuticular suturing and that does not require extra work or devices to fix the ends of the thread.

The raised problem is solved by the invention as claimed in claim 1.

The surgical thread of the invention is characterised in that: the barbs have flexible and elastic ends; the thread is devoid of a needle at its free end opposite the needle end; the first plurality of the barbs is arranged along a substantially greater proportion of the length of the thread than the second plurality of the barbs; and the second plurality of barbs is grouped adjacent the free end of the thread to resist entry of the free end of the thread into the tissue. Therefore, the thread can be threaded through the tissue, for example using the continuous method. The second plurality of barbs resist the free end of the thread pulling through the tissue. The tissue can be gathered along the thread, and the first plurality of barbs resist the tissue sliding back along the thread. The thread therefore allows one to obtain uniform moderate corrugation of the skin along the length of the wound and accordingly to obtain a shorter scar.

The barbs may be arranged in one position around the thread, or may be arranged sequentially in two, three or four positions around the thread.

Preferably, the distance by which the end of each barb protrudes from the thread is not less than the diameter of the thread. Preferably, the interval between the barbs is not less than 1.5 times the diameter of the thread. The barbs may be formed by incisions in the thread, or may be provided by needles projecting from collars fixed to the thread

Specific embodiments of the invention will now be described, purely by way of example, with reference to the accompanying drawings, in which:

| | |
|---|---|
| Figs. 1 to 3 | show three embodiments of surgical thread for plastic surgery operations; |
| Fig. 4 | shows a wound sutured with the use of surgical thread without additional fixation of its ends; and |
| Fig. 5 | shows a wound sutured by a continuous intradermic suture with a shortened scar. |

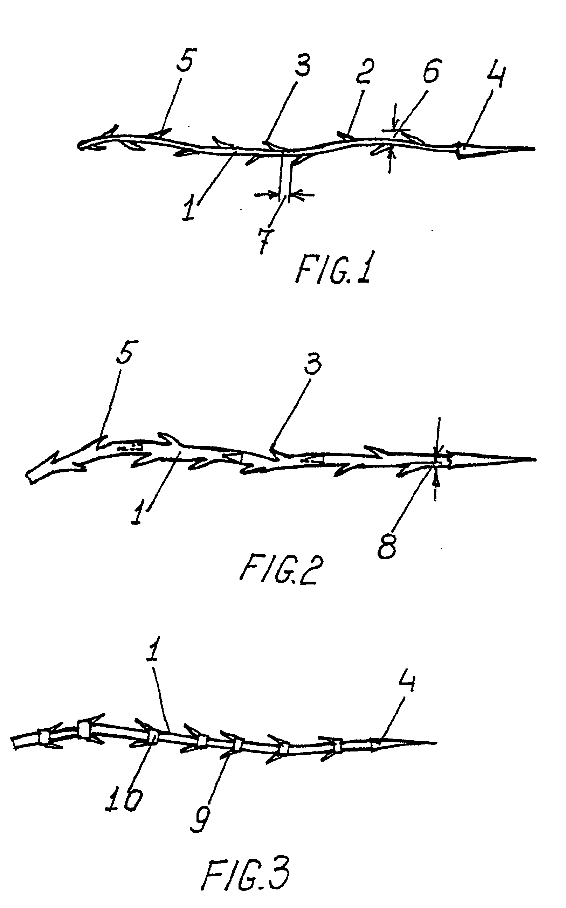

Referring to Figs. 1 to 3, each surgical thread 1 for plastic surgery operations has inclined protrusions 2,5 with sharpened conical barbs 3 arranged sequentially along its length. Most of the protrusions 2 are oriented in a direction opposite to the pull of the thread by a needle 4 at one end of the thread 1. However, a few of the protrusions 5 at the opposite end of the thread 1 are oriented in a direction opposite to that of the protrusions 2. The elevation 6 of the ends of the protrusions above the thread is less than the diameter of the thread, and the interval 7 between the protrusions is not be less than 1.5 times the diameter of the thread. In the thread 1 of Fig. 1, the protrusions 2,5 are arranged in two positions around the thread 1. In the thread 1 of Fig. 2, the protrusions are arranged in four positions around the thread 1 and are formed by incisions into the thread 1. In this case, the size of the base 8 of each incision is preferably equal to 1/3 of the diameter of the thread. In the thread of Fig. 3, the protrusions are formed by needles 9 fixed to the thread 1 by collars 10. The protrusions are formed so as to preserve the rupture strength of the thread with the provision of flexibility-elasticity of the ends of the protrusions and good engagement with hypodermic tissues preventing reverse displacement of the thread.

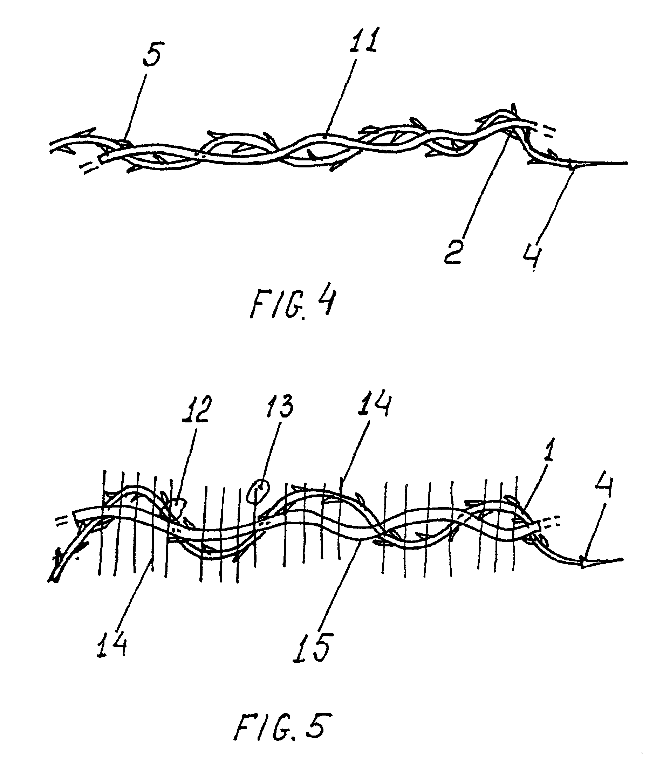

A method of suturing a wound with a subcuticular continuous suture 11 is shown on Fig. 4. The thread 1 with unidirectional penetrability can freely penetrate through hypodermic tissue in the direction of the needle 4 and on exit is prevented from reverse sliding by the ends 3 of protrusions 2. Furthermore, the other end of the thread with the protrusions 5 is prevented from slipping under the skin. During the removal of the suture the thread 1 is pulled back and cut off in front of the protrusions 5 and then it is freely pulled to the skin surface. Therefore, the thread provides the quality of suture of wound without additional fixation at the ends, thus shortening the time of operation.

A method of use of the surgical thread is shown in Fig. 5 so as to shorten of the operational scar. The skin is pierced by the needle 4 to which the thread 1 is fixed, and the thread is inserted into the hypodermic area and an intradermic continuous suture is accomplished. At each exit of the needle, for example, 12,13, the thread is pulled up into the opposite direction, the protrusions 2 by their ends 3 carry away the hypodermic tissue and gather the skin, forming uniform skin folds 14. As a result a scar 15 that is shortened in length with uniform gathering of the skin is obtained, which gives a good aesthetic effect. The skin's gathers straighten in the course of time.

## Claims

1. A surgical thread (1) for use in plastic surgery operations, the thread being formed of metallic, polymeric or biological material, the thread havin a needle (4) at one end thereof and a sequence of protruding, conical barbs (2,5) with sharpened flexible and elastic ends arranged along its length, a first plurality of the barbs (2) being inclined in a direction away from the needle-end of the thread to permit the thread to be drawn by the needle through a patient's tissue but resist sliding of the thread through the tissue in the opposite direction, and a second plurality of the barbs (5) being inclined in a direction towards the needle-end of the thread;
**characterised in that**:
the thread is devoid of a needle at its free end opposite the needle end;
the first plurality of the barbs is arranged along a substantially greater proportion of the length of the thread than the second plurality of the barbs; and
the second plurality of barbs, not less than three, is grouped adjacent the free end of the thread to resist entry of the free end of the thread into the tissue.

2. A surgical thread according to claim 1, wherein the barbs are arranged in one position around the thread.

3. A surgical thread according to claim 1, wherein the barbs are arranged sequentially in two, three or four positions around the thread.

4. A surgical thread according to any preceding claim, wherein the distance (6) by which the end of each barb protrudes from the thread is not less than the diameter of the thread.

5. A surgical thread according to any preceding claim, wherein the interval (7) between the barbs is not less than 1.5 times the diameter of the thread.

6. A surgical thread according to any preceding claim, wherein the barbs are formed by incisions in the thread.

7. A surgical thread according to any of claims 1 to 5, wherein the barbs are provided by needles (9) projecting from collars (10) fixed to the thread.

## Patentansprüche

1. Chirurgischer Faden (1) zur Verwendung bei schönheitschirurgischen Operationen, wobei der Faden aus metallischem, polymerischem oder biologischem Material gebildet ist, wobei der Faden an einem seiner Enden eine Nadel (4) und eine Sequenz vorstehender, konischer Widerhaken (2, 5) mit geschärften flexiblen und elastischen Enden aufweist, die entlang seiner Länge angeordnet sind, wobei eine erste Vielzahl der Widerhaken (2) in eine Richtung weg von dem Nadelende des Fadens geneigt sind, damit der Faden mittels der Nadel durch ein Gewebe eines Patienten gezogen werden kann, jedoch einer Verschiebung des Fadens durch das Gewebe in der entgegensetzten Richtung widersteht, und eine zweite Vielzahl von Widerhaken (5) in Richtung des Nadelendes des Fadens geneigt ist,
**dadurch gekennzeichnet, dass**
der Faden an seinem dem Nadelende entgegensetzten freien Ende keine Nadel aufweist, und
die erste Vielzahl der Widerhaken entlang eines im Wesentlichen größeren Teils der Länge des Fadens angeordnet ist, als die zweite Vielzahl der Widerhaken, und
die zweite Vielzahl von Widerhaken, nicht weniger als drei, angrenzend an das freie Ende des Fadens gruppiert sind, um einem Eintritt des freien Endes des Fadens in das Gewebe einen Widerstand zu leisten.

2. Chirurgischer Faden nach Anspruch 1, bei dem die Widerhaken in einer Position um den Faden angeordnet sind.

3. Chirurgischer Faden nach Anspruch 1, bei dem die Widerhaken sequenziell in zwei, drei oder vier Positionen um den Faden angeordnet sind.

4. Chirurgischer Faden nach einem der vorhergehenden Ansprüche, bei dem der Abstand (6), den das Ende jedes Widerhakens von dem Faden vorsteht, nicht geringer ist, als der Durchmesser des Fadens.

5. Chirurgischer Faden nach einem der vorhergehenden Ansprüche, bei dem das Intervall (7) zwischen den Widerhaken nicht weniger als das 1,5-fache des Durchmessers des Fadens beträgt.

6. Chirurgischer Faden nach einem der vorhergehenden Ansprüche, bei dem die Widerhaken durch Einschnitte in dem Faden gebildet sind.

7. Chirurgischer Faden nach einem der Ansprüche 1 bis 5, bei dem die Widerhaken von Nadeln (9) gebildet werden, die von Ringen (10) vorstehen, die an dem Faden befestigt sind.

## Revendications

1. Fil chirurgical (1) à utiliser dans des opérations de chirurgie plastique, le fil étant réalisé en une matière métallique, polymère ou biologique, le fil comportant une aiguille (4) à une de ses extrémités et une succession de barbes coniques faisant saillie (2, 5) comprenant des extrémités pointues, flexibles et élastiques arrangées sur leur longueur, un premier ensemble de barbes (2) étant incliné dans la direction s'écartant de l'extrémité du fil comportant une aiguille pour permettre au fil d'être tiré par l'aiguille à travers le tissu d'un patient, mais pour lui permettre de résister au glissement à travers le tissu dans la direction opposée, et un deuxième ensemble de barbes (5) étant incliné dans la direction s'étendant vers l'extrémité du fil comportant une aiguille ;
**caractérisé en ce que**
le fil est exempt d'aiguille à son extrémité libre opposée à l'extrémité comportant une aiguille ;
le premier ensemble de barbes est arrangé sur une proportion de la longueur du fil essentiellement supérieure à celle du deuxième ensemble de barbes ; et
le deuxième ensemble de barbes, qui n'en compte pas moins de trois, est regroupé en position adjacente à l'extrémité libre du fil pour conférer une résistance à l'entrée de l'extrémité libre du fil dans le tissu.

2. Fil chirurgical selon la revendication 1, dans lequel les barbes sont arrangées dans une position entourant le fil.

3. Fil chirurgical selon la revendication 1, dans lequel les barbes sont arrangées de manière séquentielle dans deux, trois ou quatre positions autour du fil.

4. Fil chirurgical selon l'une quelconque des revendications précédentes, dans lequel la distance (6) sur laquelle l'extrémité de chaque barbe fait saillie par rapport au fil n'est pas inférieure au diamètre du fil.

5. Fil chirurgical selon l'une quelconque des revendications précédentes, dans lequel l'intervalle (7) entre les barbes n'est pas inférieur à 1,5 fois le diamètre du fil.

6. Fil chirurgical selon l'une quelconque des revendications précédentes, dans lequel les barbes sont formées par des incisions pratiquées dans le fil.

7. Fil chirurgical selon l'une quelconque des revendications 1 à 5, dans lequel les barbes sont fournies par des aiguilles (9) faisant saillie par rapport à des bagues (10) fixées au fil.
